# FASCICULE DE BREVET EUROPEEN

(11) **EP 3 030 205 B1**
(45) Date de publication et mention de la délivrance du brevet: **13.06.2018**
(21) Numéro de dépôt: 14747599.0
(22) Date de dépôt: 30.07.2014
(51) Int. Cl.: A61F 13/00, A61F 13/02, A61F 15/00

(54) **DISPOSITIF APPLICATEUR DE PANSEMENT INTERFACE**
VORRICHTUNG ZUM AUFBRINGEN EINES SCHNITTSTELLENVERBANDES
DEVICE FOR APPLYING AN INTERFACE DRESSING

(30) Priorité: 05.08.2013 FR 1357785
(43) Date de publication de la demande: 15.06.2016
(73) Titulaire: Urgo Recherche Innovation et Développement, 21300 Chenove (FR)
(72) Inventeur: AMEY, Anne-Sophie, 21270 PERRIGNY SUR L'OGNON (FR); DESMAISON, Nadège, F-21110 Tart le Haut (FR)
(74) Mandataire: Hirsch & Associés
(86) Numéro de dépôt international: PCT/EP2014/066405
(87) Numéro de publication internationale: WO 2015/018720

(56) Documents cités:
- EP-A1- 2 380 539
- WO-A1-2013/059600
- WO-A2-2011/081810

## Description

La présente invention concerne un dispositif applicateur de pansement interface comprenant des protecteurs pelables et au moins une ailette d'application pelable. L'invention concerne également un procédé d'application d'un tel pansement interface sur un patient.

Dans le domaine du traitement des plaies, il est connu d'utiliser un pansement destiné à être appliqué directement sur une plaie en assurant une interface entre ladite plaie et une éventuelle compresse absorbante disposée après l'application dudit pansement, afin d'absorber les éventuels exsudats émanant de la plaie. Un tel pansement est habituellement désigné par le terme de « pansement interface ».

Parmi les pansements interface connus, on peut citer par exemple le produit commercialisé par la société Laboratoires URGO sous la dénomination Urgotul®.

Pour la protection et l'application des pansements interface, il est connu de FR 2 783 412 A1 un pansement interface dont chacune des faces de contact est recouverte par un film polyester. Un premier film polyester peut être retiré de la première face pour permettre l'application du pansement interface du côté de la première face sur la plaie du patient. Après application du pansement interface, le deuxième film polyester est retiré de la deuxième face pour permettre l'application d'une compresse ou d'un tampon absorbant les exsudats de la plaie au travers du pansement interface. Une telle application du pansement interface sur la peau est complexe si l'on veut éviter toute contamination par contact des doigts de l'utilisateur avec le pansement interface. Cette application du pansement interface reste complexe même dans le cas où l'utilisateur porte des gants, car le pansement interface peut adhérer aux gants.

Il est connu de WO 2008/145884 A1 un autre dispositif de protection d'un pansement interface consistant en un unique film polyester recouvrant les deux faces du pansement interface. Le film polyester peut être retiré de la première face au moyen d'une portion libre pour faciliter l'application du pansement interface sur la peau ou la plaie du patient. Toutefois, une application optimale du pansement reste complexe du fait que le film polyester recouvre totalement la deuxième face du pansement interface. Ceci limite en effet les possibilités d'adaptation du pansement interface au relief de la zone où il doit être appliqué. En outre, l'application du pansement interface, après retrait, même partiel, du film polyester de la deuxième face, ne permet pas d'éviter tout risque de contamination par des doigts de l'utilisateur.

WO2013059600 A1 concerne le domaine des couches protectrices qui sont placés sur un autre objet, y compris le corps humain, et adhérant à celui-ci, au moins temporairement, par l'intermédiaire d'un adhésif.

Or un pansement interface étant plus fragile et plus souple qu'un pansement classique, c'est-à-dire, un pansement à une seule face de contact, une application non adaptée d'un pansement interface peut entrainer une altération du pansement interface.

Il existe donc un besoin pour un pansement interface amélioré dont la manipulation est simplifiée pour l'application dudit pansement interface, sur la peau ou sur une plaie d'un patient, et ce sans altérer ledit pansement interface.

Plus particulièrement, l'invention vise à fournir un dispositif applicateur de pansement interface facilitant l'application d'un pansement interface sans l'altérer.

À cette fin, la présente invention propose un dispositif applicateur de pansement interface selon la revendication 1.

Suivant des modes de réalisation préférés, l'invention comprend une ou plusieurs des caractéristiques avantageuses selon les revendications dépendantes, prises seules ou en combinaison.

D'autres caractéristiques et avantages de l'invention apparaîtront à la lecture de la description qui suit d'un mode de réalisation préféré de l'invention, donnée à titre d'exemple et en référence aux dessins annexés.
La figure 1 représente un schéma en vue transversale d'un pansement interface.
La figure 2 représente un schéma en vue de dessus d'un pansement interface.

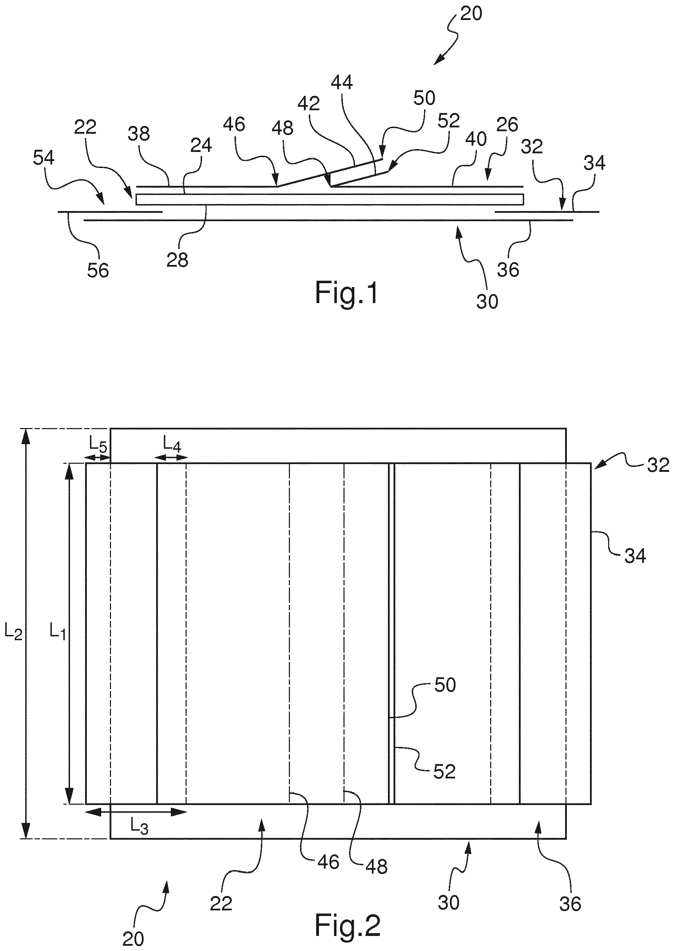

En référence à la figure 1, il est proposé un dispositif applicateur 20 de pansement interface comportant un pansement interface 22. Le pansement interface 22 comprend une première face 24 adaptée à être appliquée sur la peau ou une plaie d'un patient. Ladite première face 24 peut également comprendre dans une variante avantageuse une couche solubilisable lors de sa mise en contact avec les exsudats émanant de la plaie. Cette couche solubilisable peut comprendre notamment un principe actif choisi parmi la liste non limitative constituée des agents antibactériens, des antispetiques, des anti-douleurs, des anesthésiques, des anti-inflammatoires, des vitamines, ou encore des oligosaccharides, leurs sels ou leurs complexes. Le pansement interface 22 comporte également une deuxième face 28 opposée à la première face 24. La deuxième face 28 peut être destinée à recevoir une compresse ou un tampon absorbant les exsudats de la plaie au travers du pansement interface 22.

Pour faciliter la manipulation avant l'application du pansement interface 22, le dispositif applicateur de pansement interface 20 proposé comprend un premier et un deuxième protecteurs, 26 et 30. Ces premier et deuxième protecteurs, 26 et 30, sont respectivement disposés contre la première face 24 de contact et la deuxième face 28 de contact. Pour limiter la contamination préalable à la manipulation, les premier et deuxième protecteurs, 26 et 30, recouvrent de préférence au moins entièrement la première et la deuxième faces 24 et 28 de contact respectivement.

Pour permettre l'application du pansement interface 22 sur une plaie du patient, ou éventuellement entre une plaie et une compresse ou un tampon absorbant, les premier et deuxième protecteurs 26 et 30 sont pelables. On entend par le terme pelable, la possibilité pour un support, tel que le premier ou le deuxième protecteur 26 ou 30, d'être détaché du pansement interface 22 sur lequel il adhère sans rupture ni du support ni du pansement interface 22. Ce retrait, autrement désigné par le terme « pelage », peut notamment être réalisé par traction du support plutôt que par cisaillement, ce qui permet de limiter les risques de détérioration du pansement interface 22. La traction de pelage du protecteur 26 ou 30 du pansement interface 22 peut s'effectuer alors en tirant le protecteur 26 ou 30 et le pansement interface 22 dans deux sens opposés l'un à l'autre selon une direction perpendiculaire aux faces 24 et 28 de contact du pansement interface 22.

La première face 24 du pansement interface 22 étant destinée à être appliquée au contact avec la peau ou une plaie du patient, le premier protecteur 26 est prévu pour être pelé de la première face 24 avant l'application de la première face 24 sur la peau ou la plaie.

Pour faciliter la manipulation et l'application du pansement interface 22 après le pelage du premier protecteur 26, le dispositif applicateur de pansement interface 20 comprend en outre une ailette d'application 32 du pansement interface 22 disposée contre la deuxième face de contact 28. Cette ailette d'application 32 comprend une portion 34 s'étendant au-delà de la deuxième face 28. En s'étendant au-delà de la deuxième face 28, cette portion 34 permet la préhension sans contact direct avec le pansement interface 22. L'absence de contact entre les doigts de l'utilisateur et le pansement interface 22 permet de ne pas contaminer le pansement interface 22 et d'éviter toute adhérence entre les doigts ou les gants de l'utilisateur et le pansement interface 22 sans compliquer l'application du pansement interface 22.

Selon le mode de réalisation préféré et illustré en figure 1, le dispositif de pansement interface comprend une autre ailette d'application 54 disposée sur le côté opposé à l'ailette d'application 32. Cette autre ailette d'application 54 comprend elle aussi une portion de préhension 56 s'étendant au-delà de la deuxième face de contact 28 pour faciliter la préhension du pansement interface 22 sans contact direct. Cette deuxième ailette 54 permet une manipulation et une application encore plus aisées du pansement interface 22. En effet, la présence de l'une ou des deux ailettes d'application 32 et 54 permet de simplifier la manipulation du pansement interface 22, comme par exemple lorsque ce dernier est disposé de sorte qu'il se recouvre lui-même au moins partiellement, mais aussi de le disposer de sorte à optimiser sa pose sur une surface non plane.

Pour faciliter la manipulation et l'application du pansement interface 22, les ailettes 32 et 54 recouvrent de préférence seulement partiellement la deuxième face de contact 28. En d'autres termes, une portion de la deuxième face de contact 28 est de préférence libre de contact avec l'une ou l'autre des ailettes 32 et 54 et protégée de l'environnement extérieur par le deuxième protecteur 30. Un recouvrement seulement partiel de la deuxième face de contact 28 du pansement interface 22 par la ou les ailettes 32 et 54 permet de faciliter la manipulation et l'application du pansement interface 22 sur la peau ou la plaie du patient. En effet, dans ce cas, le pansement interface 22 est rendu plus maniable, notamment en permettant le pliage sur lui-même du pansement interface 22, que dans le cas d'un recouvrement quasi-total, voire total, de la deuxième face de contact 28 par la ou les ailettes 32 et 54.

De plus, ces ailettes 32 et 54 peuvent présenter des formes biseautées notamment au niveau des angles de la partie des ailettes 32 et 54 étant en contact avec la deuxième face de contact 28 du pansement interface 22. Ces biseaux permettent un retrait plus aisé des ailettes 32 et 54 une fois le pansement interface 22 disposé au contact de la peau ou de la plaie du patient.

Les ailettes 32 et 54 sont pelables pour permettre leur retrait du pansement interface 22 sans altérer le pansement interface 22 une fois appliqué sur la peau ou la plaie. Toutefois, le deuxième protecteur 30 est pelable indépendamment de la ou des ailettes d'application 32 et 54. Le deuxième protecteur 30 peut ainsi être retiré de la deuxième face 28 tout en laissant la ou les ailettes d'application 32 et 54 sur la deuxième face 28. Selon le mode de réalisation illustré en figure 1, le deuxième protecteur 30 peut être retiré indépendamment des ailettes d'application 32 et 54 en étant disposé sur la deuxième face de contact 28 mais au-dessus des ailettes d'application 32 et 54, c'est-à-dire en chevauchant les ailettes d'application 32 et 54. Alternativement, selon un mode de réalisation non illustré, le deuxième protecteur 30 peut être pelé indépendamment des ailettes d'application 32 et 54 en étant disposé à côté et au même niveau que les ailettes d'application 32 et 54 sur la deuxième face 28 de contact. Dans ce cas, pour limiter les contaminations, il est préféré qu'aucun espace de la deuxième face de contact 28 ne soit laissé à découvert entre le deuxième protecteur et les ailettes d'application 32 et 54. En d'autres termes, il est préféré, que les ailettes d'application 32 et 54 et le deuxième protecteur 30, tout en étant indépendantes, assurent le recouvrement entier de la deuxième face de contact 28. Dans ce cas précis, il est envisageable d'ajouter une portion d'amorce au protecteur 30 pour faciliter le retrait du deuxième protecteur 30, et ainsi limiter les risques de contaminations ou de contact avec les doigts ou les gants, notamment lors de son retrait. Préférentiellement cette portion d'amorce se trouve sur un des bords du protecteur 30.

Il est proposé un procédé d'application d'un pansement interface 22 à l'aide du dispositif applicateur de pansement interface 20 précédent. Lorsqu'un utilisateur souhaite appliquer le pansement interface 22 du dispositif applicateur de pansement interface 20 sur la peau ou une plaie d'un patient, il peut procéder de façon préférée selon les étapes suivantes : peler le premier protecteur pelable 26 ; saisir le dispositif applicateur de pansement interface 20 par la ou les portions de préhension 34 et 56 de la ou des ailettes d'application 32 et 54 ; peler le deuxième protecteur pelable 30 ; appliquer la première face 24 du pansement interface 22 sur la peau ou plaie et peler la ou les ailettes d'application 32 ou 54. Alternativement, le deuxième protecteur 30 peut être pelé après l'application de la première face 24 de contact du pansement interface 22 sur la peau ou la plaie. Le pelage du deuxième protecteur 30 s'effectue alors en maintenant la ou les portions de préhension 34 et 56 de la ou des ailettes d'application 32 et 54 sur la peau et en saisissant le deuxième protecteur 30. Néanmoins, il est préférable d'enlever le protecteur 30 préalablement à l'application du pansement interface 22 sur la peau ou la plaie du patient. En effet, le retrait du deuxième protecteur 30 permet de rendre le pansement interface 22 plus maniable, notamment en facilitant le pliage sur lui-même du pansement interface 22, que lorsque le pansement interface 22 est muni du deuxième protecteur 30. Dans tous les cas, après pelage du deuxième protecteur 30 et application du pansement interface 22 contre la peau ou la plaie du patient, une compresse ou tampon absorbant peut être disposé contre la deuxième face 28 de contact. La ou les ailettes 32 et 54 sont de préférence disposées en périphérie de la deuxième face de contact 28, c'est-à-dire sur les côtés de la deuxième face de contact 28 pour laisser libre la majeure partie de la deuxième face de contact 28 qui vient en contact avec la compresse ou le tampon absorbant. Après disposition de la compresse ou du tampon absorbant sur la deuxième face de contact 28 entre les ailettes, de préférence sur une zone de la deuxième face 28 non recouverte par les ailettes 32 et 54, les ailettes 32 et 54 peuvent être facilement pelées en prenant, par exemple, appui sur la compresse ou le tampon absorbant pour séparer la ou les ailettes d'application 32 et 54 du pansement interface 22.

En d'autres termes dans le procédé proposé, l'indépendance entre le pelage du deuxième protecteur 30 et le pelage de la ou les ailettes d'application 32 et 54 permet à l'utilisateur d'avoir un point d'appui sur la deuxième face 28 pour faciliter le pelage du deuxième protecteur 30. De plus, lorsque le deuxième protecteur 30 est pelé, la ou les ailettes d'application 32 et 54 permettent de manipuler le pansement interface 22 sans contact direct avec les faces contact 24 et 28 du pansement interface 22.

Pour faciliter le pelage du deuxième protecteur 30, une portion de préhension 36 du deuxième protecteur 30 peut s'étendre au-delà de la deuxième surface 28. En s'étendant au-delà de la deuxième face 28, cette portion 36 permet la préhension du deuxième protecteur 30 pour le pelage du deuxième protecteur 30 en écartant dans deux sens opposés l'ailette d'application 32 et la portion de préhension 36. Le pelage du deuxième protecteur 30 est ainsi facilité.

La disposition de la ou des ailettes d'application 32 et 54 sur la deuxième face 28 est avantageuse par rapport à une ailette d'application disposée sur la première face 24. Une telle ailette disposée sur la face 24 du pansement en contact avec la peau ou la plaie reste disposée entre cette première face 24 et la peau ou la plaie après application du pansement. Cette disposition de l'ailette sur la face du pansement en contact avec la peau oblige alors l'utilisateur patient à retirer l'ailette par cisaillement du pansement interface 22 en tirant la portion de préhension de l'ailette dans une direction sensiblement parallèle aux faces 24 et 28. Or, si une telle disposition de l'ailette entre la peau et le pansement peut être envisagée pour des pansements classiques à une seule face de contact tel qu'illustré en figure 12 du document EP 0 161 865 A2, le pansement interface 22, plus fragile et plus souple, présente dans un tel cas un risque d'endommagement. En effet, à cause de la fragilité du pansement interface 22, un tel cisaillement peut endommager le pansement interface 22. En particulier, le cisaillement du pansement interface 22 peut provoquer des déformations ou modifications momentanées ou durables, telles qu'une rupture, un délitement ou un fluage, du pansement interface 22 limitant l'efficacité du pansement interface 22. Le dispositif applicateur 20 proposé avec la ou les ailettes 32 et 54 sur la deuxième face de contact 28 permet lui un retrait des ailettes 32 et 54 par pelage c'est-à-dire en tirant les portions de préhension 34 et 54 pour les éloigner de la deuxième face 28 dans une direction perpendiculaire à la deuxième face 28. Un tel retrait par pelage d'une des ailettes 32 et 54 assure l'intégrité du pansement interface 22 en comparaison au retrait par cisaillement.

Par ailleurs, la disposition de l'ailette d'application sur la deuxième face 28 présente l'avantage d'un retrait moins douloureux pour le patient par rapport à une disposition de l'ailette entre le pansement interface 22 et le patient. En effet, lorsque l'ailette d'application est du côté du patient, l'ailette, lors du retrait, risque de frotter sur la peau du patient dans une zone proche de la plaie, c'est-à-dire au niveau de la peau péri-lésionnelle qui peut être fragile et sensible.

De plus, le retrait par pelage permis par la disposition des ailettes 32 et 54 sur la deuxième face 28 de contact est particulièrement plus aisé que le retrait par cisaillement lorsque le pansement est appliqué sur une zone peu accessible telle que de type concave ou cavitaire.

Le dispositif applicateur 20 proposé présente en outre l'avantage de permettre un retrait intégral de la ou des ailettes d'application 32 et 54 du pansement 22. En d'autres termes, les ailettes 32 et 54 étant pelables, après application, le pansement interface 22 peut être laissé sur la peau sans autre reste du dispositif applicateur proposé 20. Ce retrait intégral est avantageux par rapport à une ailette d'application dont une portion reste disposée sur le pansement tel que pour des ailettes de pansement classique, illustrées en figures 5 et 6 du document EP 0 161 865 A2, qui sont soit adhérentes et fixées à la peau environnant le pansement, soit retirées partiellement. En effet dans le cas du pansement interface 22, le retrait intégral de la ou des ailettes 32 et 54 assure qu'aucun obstacle ne soit présent entre la deuxième face de contact 28 et la compresse ou le tampon, i.e. toute la surface du pansement interface 22 est utilisée pour le passage des exsudats de la plaie au travers du pansement interface 22. En revanche la disposition d'ailettes restant au moins partiellement fixées sur la deuxième face de contact, soit parce que l'ailette se fixe directement à la peau, soit du fait des reliquats d'une ailette déchirée, constitue un obstacle pour le passage des exsudats de la plaie au travers du pansement interface 22. Un tel obstacle limite au moins le passage des exsudats mais aussi l'absorption desdits exsudats par la compresse ou le tampon absorbant nuisant à l'efficacité recherché pour un pansement interface.

Selon une variante illustrée sur la figure 1, le premier protecteur 26 est formé de deux pièces 38 et 40 munies chacune d'une portion d'amorce 42 ou 44 de pelage du premier protecteur 26. Les portions d'amorce 42 et 44 sont repliées vers l'extérieur du dispositif applicateur 20 par rapport au pansement interface 22, pour pouvoir être accessibles à l'utilisateur. En particulier, les portions d'amorce 42 et 44 s'étendent à partir de plis 46 et 48 jusqu'aux extrémités 50 et 52, respectivement. Les portions d'amorce 42 et 44 peuvent être repliées du même côté du premier protecteur 26, tel qu'illustré, de sorte que la portion d'amorce 42 chevauche la portion d'amorce 44. Le chevauchement des portions d'amorce 42 et 44 permet de ne pas laisser d'espace de la première face de contact à découvert entre les deux pièces 38 et 40, limitant les contaminations du pansement interface 22. A titre d'alternative, les portions d'amorce 42 et 44 peuvent être repliées vers deux côtés opposés du premier protecteur 26, c'est-à-dire que les portions d'amorce 42 et 44 sont repliées vers les pièces 38 et 40, respectivement, plutôt que vers une seule des pièces 38 et 40. Pour limiter les contaminations, il est préféré, particulièrement dans ce dernier cas, que les plis 46 et 48 soient quasiment confondus.

Selon un autre mode de réalisation non illustré, une partie du premier protecteur 26 forme une seule pièce avec une ailette d'application 32 ou 54. Dans le cas où le premier protecteur 26 est formé des deux pièces 38 et40, chacune des deux pièces 38 et 40 du premier protecteur 26 peut être formée en une seule pièce avec l'une des ailettes d'application 32 et 54. En particulier, une portion de chaque pièce 38 et 40 peut s'étendre au-delà de la première face 24 pour rejoindre l'ailette d'application du même côté, respectivement 54 et 32. Dans ce cas, lorsque la pièce 38 est pelée, celle-ci est toujours maintenue à la deuxième face 28 du pansement interface 22 via l'ailette d'application 54. De manière similaire, lorsque la pièce 40 est pelée, celle-ci est toujours maintenue à la deuxième face 28 du pansement interface 22 via l'ailette d'application 32. L'absence de recouvrement total des ailettes 32 ou 54 de la face 28 du pansement interface 22, permet une manipulation plus aisée du pansement interface 22, que ce soit dans sa préhension, mais aussi dans son application sur la peau ou la plaie du patient.

Les premier et deuxième protecteurs 26 et 30 ainsi que les ailettes 32 et 54 sont de préférence réalisés en polyoléfine, de préférence en polyester, et possèdent de préférence une épaisseur comprise entre 30 et 100 µm, de façon préférée une épaisseur comprise entre 40 et 80 µm et de façon encore plus préférée une épaisseur de 50 µm. Le pansement interface 22 peut être d'une épaisseur comprise entre 200 µm et 1 mm, de façon préférée une épaisseur comprise entre 400 et 600 µm et de façon encore plus préférée une épaisseur de 500 µm.

Selon le mode de réalisation illustré sur les figures 1 et 2, le pansement interface 22 et le deuxième protecteur 30 correspondent respectivement à un carré de côté L1 de 10 cm et un carré de côté L2 de 13 cm. Selon ce même mode de réalisation, les ailettes d'application 32 et 54 correspondent à un rectangle de largeur L3 de 3 cm et de longueur L1 de 10 cm. De plus, les zones de contact entre les ailettes d'application 32 et 54 et la deuxième face 28 correspondent à un rectangle de longueur L1 de 10 cm et de largeur L4 de 1 cm, les ailettes d'application 32 et 54 dépassant de la portion 36 du deuxième protecteur 30 d'une longueur L5 de 3cm.

Bien entendu, la présente invention n'est pas limitée aux exemples et au mode de réalisation décrits et représentés, mais elle est susceptible de nombreuses variantes accessibles à l'homme de l'art. Le premier protecteur 26 peut notamment être formé en une seule pièce recouvrant l'intégralité de la première face de contact 24 et comporter une seule portion d'amorce de pelage s'étendant au-delà de la première surface 24. En outre, le second protecteur 30 peut être formé de deux ou plusieurs pièces recouvrant l'intégralité de la deuxième face de contact 28 et de la ou des ailettes 32 et 54. Chacune de ces pièces pouvant présenter une ou plusieurs portions d'amorce.

## Revendications

1. Dispositif applicateur (20) de pansement interface destiné à être appliqué directement sur une plaie d'un patient en assurant une interface entre ladite plaie et une compresse ou un tampon absorbant disposé après l'application dudit pansement, comprenant :
- un pansement interface (22) comprenant une première (24) et une deuxième (28) faces de contact,
o la première face de contact (24) étant adaptée à être appliquée au contact de la peau ou de la plaie du patient ; et
o la deuxième face de contact (28) étant destinée à recevoir une compresse ou un tampon absorbant les exsudats émanant de la plaie au travers du pansement interface (22)
- une ailette pelable d'application (32, 54) du pansement interface (22), l'ailette d'application (32, 54) étant disposée contre la deuxième face de contact (28), une portion de préhension (34, 56) de l'ailette d'application (32) s'étendant au-delà de la deuxième face de contact (28) ;
- un premier (26) et un deuxième (30) protecteurs pelables disposés contre la première (24) et la deuxième (28) faces de contact, respectivement, le deuxième protecteur (30) étant disposé :
o au-dessus de l'ailette d'application (32, 54) de manière à chevaucher l'ailette d'application (32, 54) ; ou
o à côté et au même niveau que l'ailette d'application (32, 54) sur la deuxième face (28) de contact.

2. Dispositif applicateur (20) selon la revendication 1, quand le deuxième protecteur (30) est disposé au-dessus de l'ailette d'application (32, 54) de manière à chevaucher l'ailette d'application (32, 54), le premier (26) et le deuxième (30) protecteurs recouvrent entièrement la première (24) et la deuxième (28) faces de contact, respectivement.

3. Dispositif applicateur (20) selon la revendication 1 ou 2, quand le deuxième protecteur (30) est disposé au-dessus de l'ailette d'application (32, 54) de manière à chevaucher l'ailette d'application (32, 54), le deuxième protecteur (30) s'étend au-delà de la deuxième face de contact (28) à l'aide d'une portion de préhension (36).

4. Dispositif applicateur (20) selon la revendications 2 ou 3, dans lequel le deuxième protecteur (30) est formé de deux ou plusieurs pièces recouvrant l'intégralité de la deuxième face de contact (28) et de l'ailette (32, 54).

5. Dispositif applicateur (20) selon la revendication 4, dans lequel chacune de ces pièces du deuxième protecteur (30) présente une ou plusieurs portions d'amorce.

6. Dispositif applicateur (20) selon la revendication 1, quand le deuxième protecteur (30) est disposé à côté et au même niveau que l'ailette d'application (32, 54) sur la deuxième face (28) de contact, l'ailette d'application (32, 54) et le deuxième protecteur (30), tout en étant indépendantes, assurent le recouvrement entier de la deuxième face de contact (28).

7. Dispositif applicateur (20) selon la revendication 6, dans lequel le deuxième protecteur (30) comprend une portion d'amorce pour faciliter le retrait du deuxième protecteur (30), et ainsi limiter les risques de contaminations lors du retrait.

8. Dispositif applicateur (20) selon la revendication 7, dans lequel la portion d'amorce est disposée sur un des bords du protecteur (30).

9. Dispositif applicateur (20) selon l'une quelconque des revendications précédentes, dans lequel l'ailette (32, 54) de la deuxième face de contact (28) recouvre partiellement la deuxième face de contact (28) pour faciliter la manipulation et l'application du pansement interface (22).

10. Dispositif applicateur (20) selon l'une des revendications 1 à 6, dans lequel une partie (38, 40) du premier protecteur (26) forme une seule pièce avec l'ailette d'application (32, 54).

11. Dispositif applicateur (20) selon l'une quelconque des revendications précédentes, comprenant une autre ailette pelable d'application (54) avec une portion de préhension (56), les ailettes d'application (32, 54) étant disposées sur deux côtés opposés de la deuxième face de contact (28), la portion de préhension (34, 56) de chacune des ailettes d'application (32, 54) s'étendant au-delà de la deuxième face de contact (28).

12. Dispositif applicateur (20) selon la revendication 11, dans le cas où le premier protecteur (26) est formé des deux pièces (38, 40), chacune des deux pièces (38, 40) du premier protecteur (26) est formée en une seule pièce avec l'une des ailettes d'application (32, 54) du même côté.

13. Dispositif applicateur (20) selon l'une quelconque des revendications précédentes, dans lequel le premier protecteur (26) comporte une portion d'amorce (42, 44) de pelage dudit premier protecteur (26).

14. Dispositif applicateur (20) selon la revendication 13, dans lequel le premier protecteur (26) est formé de deux pièces (38, 40) munies chacune d'une portion d'amorce (42, 44) de pelage repliée sur le reste du premier protecteur (26) vers l'extérieur du dispositif (20) par rapport au pansement interface (22).

15. Dispositif applicateur (20) selon la revendication 13, dans lequel le premier protecteur (26) est formé en une seule pièce recouvrant l'intégralité de la première face de contact (24) et comporte une seule portion d'amorce de pelage s'étendant au-delà de la première surface (24).

## Patentansprüche

1. Aufbringungsvorrichtung (20), die zum Aufbringen eines Schnittstellenverbands unmittelbar auf eine Wunde eines Patienten bestimmt ist, wobei sie, nach dem Aufbringen des Verbands, eine Zwischenfläche zwischen der Wunde und einer Kompresse oder einem absorbierenden Kissen gewährleistet, umfassend:
- einen Schnittstellenverband (22), der eine erste (24) und eine zweite (28) Kontaktfläche umfasst, wobei
o die erste Kontaktfläche (24) ausgebildet ist, um im Kontakt mit der Haut oder der Wunde des Patienten aufgebracht zu werden; und
o die zweite Kontaktfläche (28) dazu bestimmt ist, eine Kompresse oder ein, die Wundexsudate absorbierendes Kissen durch den Schnittstellenverband (22) aufzunehmen
- eine abziehbare Aufbringungslasche (32, 54) des Schnittstellenverbands (22), wobei die Aufbringungslasche (32, 54) der zweiten Kontaktfläche (28) gegenüberliegend angeordnet ist, und wobei ein Griffabschnitt (34, 56) der Aufbringungslasche (32) sich über die zweite Kontaktfläche (28) hinaus erstreckt;
- eine erste (26) und eine zweite (30) abziehbare Schutzeinrichtung, die jeweils der ersten (24) und der zweiten Kontaktfläche (28) gegenüberliegend angeordnet sind, wobei die zweite Schutzeinrichtung (30) folgendermaßen angeordnet ist:
o oberhalb der Aufbringungslasche (32, 54), so dass sie die Aufbringungslasche (32, 54) überlappt; oder
o neben der Aufbringungslasche (32, 54) und in gleicher Höhe damit, auf der zweiten Kontaktfläche (28) .

2. Aufbringungsvorrichtung (20) nach Anspruch 1, wobei, wenn die zweite Schutzeinrichtung (30) oberhalb der Aufbringungslasche (32, 54) angeordnet ist, so dass sie die Aufbringungslasche (32, 54) überlappt, die erste (26) und die zweite (30) Schutzeinrichtung jeweils die erste (24) und die zweite (28) Kontaktfläche vollständig überdecken.

3. Aufbringungsvorrichtung (20) nach Ansprüchen 1 oder 2, wobei, wenn die zweite Schutzeinrichtung (30) oberhalb der Aufbringungslasche (32, 54) angeordnet ist, so dass sie die Aufbringungslasche (32, 54) überlappt, die zweite (30) Schutzeinrichtung (30) sich mithilfe eines Griffabschnitts (36) über die zweite Kontaktfläche (28) hinaus erstreckt.

4. Aufbringungsvorrichtung (20) nach Ansprüchen 2 oder 3, wobei die zweite Schutzeinrichtung (30) aus zwei oder mehreren Teilen gebildet ist, welche die Gesamtheit der zweiten Kontaktfläche (28) und der Lasche (32, 54) überdecken.

5. Aufbringungsvorrichtung (20) nach Anspruch 4, wobei jedes der Teile der zweiten Schutzeinrichtung (30) einen oder mehrere Anfangsabschnitte aufweist.

6. Aufbringungsvorrichtung (20) nach Anspruch 1, wobei, wenn die zweite Schutzeinrichtung (30) neben der Aufbringungslasche (32, 54), und in gleicher Höhe damit, auf der zweiten Kontaktfläche (28) angeordnet ist, die Aufbringungslasche (32, 54) und die zweite Schutzeinrichtung (30) unabhängig voneinander die vollständige Überdeckung der zweiten Kontaktfläche (28) gewährleisten

7. Aufbringungsvorrichtung (20) nach Anspruch 6, wobei die zweite Schutzeinrichtung (30) einen Anfangsabschnitt umfasst, um die Entfernung der zweiten Schutzeinrichtung (30) zu erleichtern und somit bei der Entfernung die Risiken einer Kontamination zu begrenzen.

8. Aufbringungsvorrichtung (20) nach Anspruch 7, wobei der Anfangsabschnitt an einem der Ränder der Schutzeinrichtung (30) angebracht ist.

9. Aufbringungsvorrichtung (20) nach einem der vorhergehenden Ansprüche, wobei die Lasche (32, 56) der zweiten Kontaktfläche (28) die zweite Kontaktfläche (28) teilweise überdeckt, um das Handhaben und die Aufbringung des Schnittstellenverbands (22) zu erleichtern.

10. Aufbringungsvorrichtung (20) nach einem der Ansprüche 1 bis 6, wobei ein Teil (38, 40) der ersten Schutzeinrichtung (26) mit der Aufbringungslasche (32, 54) einstückig ausgebildet ist.

11. Aufbringungsvorrichtung (20) nach einem der vorhergehenden Ansprüche, umfassend eine weitere abziehbare Aufbringungslasche (54) mit einem Griffabschnitt (56), wobei die Aufbringungslaschen (32, 54) auf zwei entgegengesetzte Seiten der zweiten Kontaktfläche (28) angeordnet sind, und wobei der Griffabschnitt (34, 56) von jeder der Aufbringungslaschen (32, 54) sich über die zweite Kontaktfläche (28) hinaus erstreckt.

12. Aufbringungsvorrichtung (20) nach Anspruch 11, wobei, wenn die erste Schutzeinrichtung (26) aus zwei Teilen (38, 40) gebildet ist, jedes der zwei Teile (38, 40) der ersten Schutzeinrichtung (26) mit einer der Aufbringungslaschen (32, 54) an der gleichen Seite einstückig ausgebildet ist.

13. Aufbringungsvorrichtung (20) nach einem der vorhergehenden Ansprüche, wobei die erste Schutzeinrichtung (26) einen Anfangsabschnitt (42, 44) zum Abziehen der ersten Schutzeinrichtung (26) aufweist

14. Aufbringungsvorrichtung (20) nach Anspruch 13, wobei die erste Schutzeinrichtung (26) aus zwei Teilen (38, 40) gebildet ist, von denen jedes mit einem, im Verhältnis zum Schnittstellenverband (22), über den Rest der ersten Schutzeinrichtung (26), nach außen zur Aufbringungsvorrichtung (20) hin gefalteten, abziehbaren Anfangsabschnitt (42, 44) versehen ist.

15. Aufbringungsvorrichtung (20) nach Anspruch 13, wobei, die erste Schutzeinrichtung (26) aus einem Teil gebildet ist, dass die Gesamtheit der ersten Kontaktfläche (24) überdeckt, und einen einzigen abziehbaren Anfangsabschnitt aufweist, der sich über die erste Oberfläche (24) hinaus erstreckt.

## Claims

1. An interface dressing applicator device (20) for direct application to a wound of a patient by providing an interface between said wound and an absorbent compress or a pad disposed after application of said dressing, comprising:
- an interface dressing (22) comprising a first (24) and a second (28) contact surface,
• the first contact surface (24) being suitable for being applied in contact with the skin or a wound of a patient; and
• the second contact surface (28) being intended to receive a compress or a aipad absorbing the exudates emanating from the wound through the interface dressing (22)
- a peelable applicator tab (32, 54) of the interface dressing (22), the applicator tab (32, 54) being disposed against the second contact surface (28), a gripping portion (34, 56) of the applicator tab (32) extending beyond the second contact surface (28);
- a first (26) and a second (30) peelable protectors disposed against the first (24) and second (28) contact surfaces, respectively, the second protector (30) being disposed:
• above the applicator tab (32, 54) so as to overlap the applicator tab (32, 54); or
• next to and at the same level as the applicator tab (32, 54) on the second contact surface (28).

2. The applicator device (20) according to claim 1, when the second protector (30) is disposed above the applicator tab (32, 54) so as to overlap the applicator tab (32, 54), the first (26) and second (30) protectors completely cover the first (24) and second (28) contact surfaces, respectively.

3. The applicator device (20) according to claim 1 or 2, when the second protector (30) is disposed above the applicator tab (32, 54) so as to overlap the applicator tab (32, 54), the second protector (30) extends beyond the second contact surface (28) with the aid of a gripping portion (36).

4. The applicator device (20) according to claim 2 or 3, wherein the second protector (30) is formed of two or more pieces covering the entirety of the second contact surface (28) and of the tab (32, 54).

5. The applicator device (20) according to claim 4, wherein each of said pieces of the second protector (30) has one or more starter portions.

6. The applicator device (20) according to claim 1, when the second protector (30) is disposed adjacent to and at the same level as the applicator tab (32, 54) on the second contact surface (28), the applicator tab (32, 54) and the second protector (30), while still being independent, providing complete covering of the second contact surface (28).

7. The applicator device (20) of claim 6, wherein the second protector (30) comprises a starter portion to facilitate removal of the second protector (30), and thereby limit the risk of contaminations upon removal.

8. The applicator device (20) of claim 7, wherein the starter portion is disposed on one edge of the protector (30).

9. The applicator device (20) according to any one of the preceding claims, wherein the tab (32, 54) of the second contact surface (28) partially overlaps the second contact surface (28) to facilitate handling and application of the interface dressing (22).

10. The applicator device (20) according to one of claims 1 to 6, wherein a portion (38, 40) of the first protector (26) is of a one-piece construction with the applicator tab (32, 54).

11. The applicator device (20) according to any one of the preceding claims, comprising another peelable applicator tab (54) with a gripping portion (56), the applicator tabs (32, 54) being disposed on two opposite sides of the second contact surface (28), the gripping portion (34, 56) of each of the applicator tabs (32, 54) extending beyond the second contact surface (28).

12. The applicator device (20) according to claim 11, in the case where the first protector (26) is formed of the two parts (38, 40), each of the two parts (38, 40) of the first protector (26) is formed in one piece with one of the applicator tabs (32, 54) on the same side.

13. The applicator device (20) according to any one of the preceding claims, wherein the first protector (26) has a starter portion (42, 44) for peeling said first protector (26).

14. The applicator device (20) according to claim 13, wherein the first protector (26) is formed of two parts (38, 40) each provided with a portion of peeling starter (42, 44) folded over the remainder of the first protector (26) outwardly of the device (20) relative to the interface dressing (22).

15. The applicator device (20) of claim 13, wherein the first protector (26) has a one-piece construction covering the entire first contact surface (24) and has a single peel starter portion extending beyond the first surface (24).
